# EUROPEAN PATENT SPECIFICATION

(11) **EP 1 575 563 B1**
(45) Date of publication and mention of the grant of the patent: **14.02.2007**
(21) Application number: 03796731.2
(22) Date of filing: 05.12.2003
(51) Int. Cl.: A61K 9/16, A61K 31/155

(54) **NON-HYGROSCOPIC FORMULATION COMPRISING A HYDROSCOPIC DRUG**
NICHT-HYGROSKOPISCHE FORMULIERUNG, DIE EINEN HYGROSKOPISCHEN WIRKSTOFF ENTHÄLT
FORMULATION A CARACTERE NON-HYGROSCOPIQUE COMPRENANT UN PRINCIPE ACTIF HYDROSCOPIQUE

(30) Priority: 19.12.2002 US 435022 P; 19.12.2002 US 435147 P; 19.12.2002 US 435422 P
(43) Date of publication of application: 21.09.2005
(73) Proprietor: Pharmacia Corporation, Chesterfield, MO 63017-1732 (US)
(72) Inventor: CZYZEWSKI, Ann M., c/o Pfizer Global R & D, Chesterfield, MO 63017-1732 (US); GAO, Danchen Pfizer Global Research&Development, Chesterfield, MO 63017-1732 (US)
(74) Representative: Bridle, Andrew Barry
(86) International application number: PCT/US2003/038792
(87) International publication number: WO 2004/060352

(56) References cited:
- WO-A-01/72703
- US-A- 4 835 187
- US-A- 5 582 837
- US-A- 5 939 100
- US-A1- 2002 143 058
- DATABASE WPI Week 8941 Derwent Publications Ltd., London, GB; AN 1990-309040 XP002282117 "Sustained release compositions, comprising water-soluble active substance, water soluble macromolecular compound and water insoluble macromolecular compound" & JP 02 218621 A (NIPPON CHEMIPHAR CO) 31 August 1990 (1990-08-31)

## Description

The present invention relates to pharmaceutical compositions comprising a hygroscopic and/or deliquescent drug selected from S-[2-[(1-iminoethyl)amino]ethyl] -2-methyl-L-cysteine and its dihydrochloride salt, more particularly to such compositions suitable as intermediates for further processing.

### BACKGROUND OF THE INVENTION

The sorption of moisture by drugs can create significant problems. In presence of moisture a solid drug substance can become hydrated and/or convert to a new crystal form. Moisture sorption can also adversely affect release rate of a substance from formulation, shelf life of a formulation, and handling and processing properties of the substance itself. Hygroscopic and/or deliquescent drugs, by definition, are prone to experiencing these adverse effects when exposed to environments with even moderate humidity. Thus, it is usually imperative to control moisture sorption during formulation and storage.

Manufacturing plant alterations, such as installation of machinery to reduce humidity within a manufacturing plant, have been used to limit exposure of hygroscopic and/or deliquescent drugs to humid conditions during production and packaging. However, such alterations are disadvantageous in being costly and unreliable in effectiveness. Furthermore, alterations in manufacturing conditions do very little to protect a hygroscopic and/or deliquescent drug during subsequent storage and transport.

Hygroscopic and/or deliquescent drugs also pose problems that do not directly result from interactions with humid environments. For example, U.S. Patent No. 5,037,698 to Brunel reports that when a hygroscopic and/or deliquescent drug is incorporated into a gelatin capsule, a commonly used dosage form, the drug tends to absorb moisture from the capsule wall, leaving the capsule in a brittle or deformed state, susceptible to breakage and leakage.

U.S. Patent No. 5,225,204 to Chen *et al.,* describes compositions comprising a complex of the hygroscopic drug levothyroxine sodium and a water soluble polyvinylpyrrolidone which complex is adsorbed on a cellulose compound. Such compositions are said to be stable in humid conditions.

U.S. Patent No. 4,223,006 to Taskis discloses particles consisting of the hygroscopic compound clavulanic acid dispersed in a solution of non-aqueous solvent and polymeric binder (*e.g.* ethylcellulose and polyvinyl acetate phthalate of low water vapor permeability). The particles are prepared by depositing a binder on the salt of calvulanic acid under substantially anhydrous conditions and are said to absorb significantly less moisture when subjected to humid conditions than unformulated clavulanic acid particles. Above-cited U.S. Patent No. 4,223,006 teaches that a disintegrant, such as microcrystalline cellulose, can be blended with the particles after the dispersion has formed.

U.S. Patent No. 6,204,255 to Klokkers discloses non-deliquescent solid dispersions consisting of the hygroscopic and deliquescent drug sodium valproate and expensive cyclodextrins. While the disclosed dispersions, when subjected to humid conditions, absorbed less moisture than unformulated sodium valproate, the dispersions still exhibited significant moisture absorption at 75% relative humidity.

Therefore, a need exists for acceptably non-hygroscopic solid compositions comprising a hygroscopic and/or deliquescent drug that can readily be formulated as convenient dosage forms and that are suitable for large-scale manufacture.

Illustratively, such a need exists where the drug is an inhibitor of inducible nitric oxide synthase (hereinafter referred to as a "iNOS inhibitor"), a class of therapeutic agents useful in treatment of a wide range of inflammatory conditions and other disorders mediated by iNOS. In particular, such a need exists where the drug is a preferential, selective or specific iNOS inhibitor, *i.e.*, having significantly greater inhibitory effect on inducible forms of the nitric oxide synthase enzyme than on constitutive forms of the enzyme, such as a drug selected form S-[2-[(1-iminoethyl)amino]ethyl]-2-methyl-L-cysteine and its dihydrochloride salt.

### SUMMARY OF THE INVENTION

Accordingly, the present invention provides a solid particulate composition comprising a hygroscopic and/or deliquescent drug selected from S-[2-[(1-iminoethyl)amino]ethyl]-2-methyl-L-cysteine and its dihydrochloride salt, and at least one non-hygroscopic cellulosic polymer wherein the drug and the polymer are in intimate association and the composition is acceptably non-hygroscopic.

Preferably, the composition is flowable and/or compressible.

Optionally, the composition comprises a filler wherein the filler is preferably hygroscopic and/or deliquescent.

The drug is an iNOS inhibitor,

In one embodiment, the solid particulate composition of the present invention may be prepared by spray drying an aqueous liquid having the drug and the at least one polymer dispersed therein.

The term "hygroscopic" as used herein refers to materials, such as drugs or pharmaceutical excipients, that absorb significant amounts of atmospheric moisture when exposed to conditions of normal ambient relative humidity (RH), for example 10-50% RH. The term "deliquescent" refers to drugs or excipients that tend to undergo gradual dissolution and/or liquefaction due to attraction and/or absorption of moisture from air when exposed to these conditions. Those skilled in the art will appreciate that over the usual range of ambient temperatures used in drug formulation, hygroscopicity and the state of deliquescence are largely temperature independent, and that there are varying degrees of hygroscopicity and deliquescence. Thus, for example, adverbs such as "very," "slightly," or "extremely" sometimes precede the words "hygroscopic" or "deliquescent" in descriptions of drugs or excipients in order to indicate the amount of moisture a particular drug or excipient tends to absorb in humid climates or the degree to which a particular drug or excipient tends to dissolve and/or liquefy due to attraction and/or absorption of moisture from humid air. As used herein, "hygroscopic" refers to drugs or excipients that are at least slightly hygroscopic. Similarly, the term "deliquescent" herein refers to drugs or excipients that are at least slightly deliquescent.

The term "acceptably non-hygroscopic" with respect to a solid particulate composition of the invention comprising an otherwise hygroscopic and/or deliquescent drug means that the composition does not absorb substantial amounts of moisture when subjected to relatively humid conditions, for example 40-70% RH. Consequently, shelf life, flow, handling and processing properties of the composition are generally not substantially affected by exposure to such conditions.

Compositions of the invention provide a surprisingly effective solution to the moisture sorption problem associated with hygroscopic and/or deliquescent drugs. It is particularly surprising that such hygroscopic and/or deliquescent drugs can be prepared into acceptably non-hygroscopic formulation intermediates by an aqueous spray drying process. Spray drying in and of itself is advantageous in that it offers continuous processing and production conditions. Furthermore, spray drying using an aqueous dispersion (as opposed to non-aqueous solvents) is particularly advantageous in that it avoids potential chemical interaction between a non-aqueous solvent and drug, and eliminates potential toxicities associated with many non-aqueous solvents.

### BRIEF DESCRIPTION OF THE DRAWINGS

Fig. 1 shows a moisture sorption isotherm of hygroscopic Compound 1.

Fig. 2 shows equilibrium water uptake (hygroscopicity profile) by solid particulate compositions SP2- SP6 of Example 2 upon storage in a humidity chamber for a period of 120 hours.

Fig. 3 shows equilibrium water uptake by solid particulate compositions SP8 and SP10 - SP14 of Example 2 upon storage in a humidity chamber for a period of 120 hours.

Fig. 4 shows equilibrium water uptake by solid particulate compositions SP15 - SP17 of Example 5 and of matching placebo compositions P1 - P3 of Example 5 upon storage in a humidity chamber for a period of at least 400 hours.

Fig. 5 shows equilibrium water uptake by solid particulate compositions SP18 - SP22 of Example 6 upon storage in a humidity chamber for a period of 120 hours.

### DETAILED DESCRIPTION OF THE INVENTION

### Hygroscopic and/or deliquescent drug

A composition of the invention comprises a hygroscopic and/or deliquescent drug. S-[2-[(1-iminoethyl)anuno]ethyl]-2-methyl-L-cysteine is the drug used in a composition of the invention. This drug, disclosed in International Patent Publication No. WO 01/72703, is a nitric oxide synthase (NOS) inhibitor, and is believed to have value in, for example, treating inflammation and other NOS-mediated disorders, such as pain, headache and fever. S-[2-[(1-iminoethyl)amino]ethyl]-2-methyl-L-cysteine for use herein can be prepared by any suitable means, including processes described in above-cited International Patent Publication No. WO 01/72703. This compound can be used in its free base form or as a pharmaceutically acceptable salt, for example the dihydrochloride salt.

It has now been found that S-[2-[(1-iminoethyl)amino]ethyl]-2-methyl-L-cysteine and its dihydrochloride salt are extremely hygroscopic and deliquescent. It is particularly surprising that such a hygroscopic and deliquescent drug can be formulated in accordance with the present invention as an acceptably non-hygroscopic composition.

S-[2-[(1-iminoethyl)amino]ethyl]-2-methyl-L-cysteine and its dihydrochloride salt is preferably present in a composition of the invention in an amount of at least about 5%, more preferably at least about 10%, still more preferably at least about 15% and even more preferably at least about 20% by weight of the composition. For example, S-[2-[(1-iminoethyl)amino]ethyl]-2-methyl-L-cysteine and its dihydrochloride salt, is present in the instant composition at 10% to 80%, more preferably 15% to 60%, and still more preferably 20% to 40% by weight of the composition.

### Non-hygroscopic polymer

The instant composition comprises at least one non-hygroscopic polymer. The term "non-hygroscopic polymer" herein means that the polymer exhibits an equilibrium moisture uptake at 40% RH of not more than about 8%, preferably not more than about 7%, and more preferably not more than about 6%, for example about 1% to about 5%. The non-hygroscopic polymer is a cellulosic polymer, for example, hydroxypropylmethylcellulose (HPMC), hydroxypropylcellulose (HPC), hydroxyethylcellulose, methylcellulose and/or ethylcellulose.

Hydroxyethylcellulose is another preferred cellulosic polymer. Exemplary hydroxyethylcelluloses useful in the invention include those having low dynamic viscosity in aqueous media, preferably below about 400 cps, *e.g.,* below about 150 cps as measured in a 2% aqueous solution at 25°C. Hydroxyethylcellulose is available for example under the tradenames Cellosize^{™} (Amerchol) and Natrusol^{™} (Aqualon).

Preferably, the weight ratio of total polymer to S-[2-[(1-iminoethyl)amino]ethyl]-2-methyl-L-cysteine and its dihydrochloride salt, is about 1:5 to about 5:1, more preferably about 1:3 to about 3:1, still more preferably about 1:2 to about 2:1, and yet more preferably about 1:1.5 to about 1.5:1. An especially preferable polymer to drug weight ratio is about 1:1 to about 1:1.5.

In a preferred embodiment of the invention, the non-hygroscopic polymer is present in the composition in a total amount of about 10% to about 85%, more preferably about 30% to about 80%, and still more preferably about 40% to about 75% by weight of the composition.

Hydroxypropylmethyl cellulose (HPMC) is an especially preferred cellulosic polymer. A preferred HPMC is one with a low apparent dynamic viscosity, preferably below about 100 cps as measured at 20°C for a 2% aqueous solution, more preferably below about 50 cps, and still more preferably below about 20 cps, for example 3 or 5 cps. HPMC, including a grade having apparent dynamic viscosity of 3 cps, is available for example under the tradename Pharmacoat^{™} 603 (Shin-Etsu).

### Intimate Association between the drug and the polymer.

Without being bound by theory, the composition of the present invention is surprisingly non hydroscopic due in part, to the intimate association between the hygroscopic and/or deliquescent drug and the at least one non-hydroscopic polymer. As used herein, the term "the intimate association" is the association that results from, by way of example, co-dispersing the drug and the polymer in an aqueous liquid and then removing the liquid (e.g. by spray drying, evaporation, lyophilization, *etc.*) to form a solid particulate composition.

### Fillers

Compositions of the invention optionally comprise one or more fillers. The term "filler," as used herein, refers to inert materials that serve to increase the mass and/or bulk density of a composition of the invention, so that, for example, the composition can be relatively easily incorporated into a conventional dosage form, e.g., a tablet or capsule. Preferably the filler used does not adversely affect the stability and/or dissolution performance of the dispersion.

The filler itself can be non-hygroscopic or hygroscopic and/or deliquescent. Fillers of the present invention can be cellulosic or noncellulosic. For the purpose of clarity, instant fillers can be (1) cellulosic and non-hygroscopic; (2) noncellulosic and non-hygroscopic; (3) non cellulosic and hygroscopic and/or deliquescent; or (4) cellulosic and hygroscopic and/or deliquescent.

In one preferred embodiment, the filler is hygroscopic and/or deliquescent. Hygroscopic and/or deliquescent fillers include for example microcystalline cellulose, tribasic calcium phosphate, anhydrous calcium sulfate, carboxymethylcellulose calcium, carboxymethylcellulose sodium, anhydrous dextrose, fructose, anhydrous lactose, anhydrous magnesium stearate, magnesium trisilicate, maltodextrin, methylcellulose, powdered cellulose, pregelatinized starch, starch, sterilizable maize starch, compressible sugar, confectioner's sugar and the like.

Particularly preferably the filler is a hygroscopic and/or deliquescent cellulosic polymer, e.g., microcrystalline cellulose, carboxymethylcellulose sodium, carboxymethylcellulose calcium, methylcellulose or powdered cellulose. Especially preferably the filler is microcrystalline cellulose, available for example under the tradename Avicel^{™} (FMC) in various grades.

Fillers contemplated for use in the present invention illustratively include microcrystalline cellulose, lactose, calcium carbonate, carboxymethylcellulose calcium, carboxymethylcellulose sodium, dibasic calcium phosphate dihydrate, tribasic calcium phosphate, calcium sulfate, dextrose, ethyl cellulose, fructose, kaolin, magnesium carbonate, magnesium stearate, magnesium trisilicate, maltol, maltodextrin, mannitol, methyl cellulose, powdered cellulose, pregelatinized starch, starch, sterilizable maize starch, compressible sugar, confectioner's sugar and the like.

The filler is preferably present in an amount sufficient to enable the composition, once formed, to be in a flowable state, such as a powder, that can be easily incorporated into conventional dosage forms, such as tablets and capsules. The filler is more preferably present in an amount sufficient to enable the composition, once formed, to be in both a flowable and compressible state, such as a powder, that can be easily incorporated into conventional dosage forms, such as tablets and capsules. Accordingly, the filler is generally present in an amount of about 1% to about 95%, preferably about 5% to about 30% by weight of the composition. The present inventors have found that hygroscopic and/or deliquescent cellulosic polymers, such as microcrystalline cellulose, in an amount of about 20% to about 30%, by weight of the composition, are particularly well-suited for the present invention.

Various methods are known to those skilled in the art for detecting or measuring moisture absorption by a composition. An illustrative method that is convenient and easy to apply in most situations is observation and/or measurement of increase in mass of a composition upon exposure to humidity. A composition of the invention preferably exhibits an increase in mass of not more than about 10%, more preferably not more than about 7%, and even more preferably not more than about 6%, when subjected to conditions of 40% relative humidity and ambient temperatures (21-23°C) for 24 hours and/or for a time sufficient to achieve substantial equilibrium, *i.e.,* a time after which no further significant increase in mass is observed.

### Moisture sorption and handling properties

Additionally, a composition of the invention preferably will be in the form of a free-flowing powder when maintained under conditions of 40% relative humidity and ambient temperature.

### Particle size distribution

A composition of the invention is preferably in the form of a flowable powder comprising particles or granules. Preferably, such particles or granules will have a D₉₀ size, by volume, of about 20 µm to about 800 µm, preferably about 40 µm to about 500 µm, and more preferably about 50 µm to about 300 µm.

### Process for preparing an acceptably non-hygroscopic composition

A composition of the invention can be prepared by any suitable process for bringing into intimate association the drug and the non-hygroscopic polymer. A particularly preferred process for preparing a composition of the invention comprises co-dispersing the hygroscopic and/or deliquescent drug and the non-hygroscopic polymer in an aqueous liquid and then removing the liquid, for example by spray drying, evaporation, lyophilization, *etc*. In a particularly preferred embodiment, the liquid is removed by spray drying.

### Utility

Compositions of the present invention are useful in providing a hygroscopic and/or deliquescent drug in a pharmaceutically acceptable, non-hygroscopic formulation for subjects in need thereof.

The hygroscopic drug is an iNOS inhibitor. Pharmaceutically acceptable compositions of the invention are useful for treating, *inter alia,* inflammation in a subject, or for treating other nitric oxide synthase-mediated disorders such as pain, headaches or fever. For example, such compositions are useful to treat arthritis, including but not limited to rheumatoid arthritis, spondyloarthropathies, gouty arthritis, osteoarthritis, systemic lupus, erythematosus, juvenile arthritis, acute rheumatic arthritis, enteropathic arthritis, neuropathic arthritis, psoriatic arthritis, and pyogenic arthritis. Conditions in which the compositions of the present invention will provide an advantage in inhibiting NOS production from L-arginine include arthritic conditions.

Such compositions are further useful in the treatment of asthma, bronchitis, menstrual cramps (*e.g*., dysmenorrhea), premature labor, tendonitis, bursitis, skin-related conditions such as psoriasis, eczema, burns, sunburn, dermatitis, pancreatitis, hepatitis, and from post-operative inflammation including from ophthalmic surgery such as cataract surgery and refractive surgery. Such compositions are also useful to treat gastrointestinal conditions such as inflammatory bowel disease, Crohn's disease, gastritis, irritable bowel syndrome and ulcerative colitis. Such compositions are useful for the prevention or treatment of cancer, such as colorectal cancer, and cancer of the breast, lung, prostate, bladder, cervix and skin. Such compositions are useful in treating inflammation and tissue damage in such diseases as vascular diseases, migraine headaches, periarteritis nodosa, thyroiditis, aplastic anemia, Hodgkin's disease, sclerodoma, rheumatic fever, type I diabetes, neuromuscular junction disease including myasthenia gravis, white matter disease including multiple sclerosis, sarcoidosis, nephrotic syndrome, Behcet's syndrome, polymyositis, gingivitis, nephritis, hypersensitivity, swelling occurring after injury, myocardial ischemia, and the like. Such compositions are useful in the treatment of ophthalmic diseases, such as glaucoma, retinitis, retinopathies, uveitis, ocular photophobia, and of inflammation and pain associated with acute injury to the eye tissue. Of particular interest among the uses of the present inventive compositions is the treatment of glaucoma, especially where symptoms of glaucoma are caused by the production of nitric oxide, such as in nitric oxide-mediated nerve damage. Such compositions are useful in the treatment of pulmonary inflammation, such as that associated with viral infections and cystic fibrosis. Such compositions are useful for the treatment of certain central nervous system disorders, such as cortical dementias including Alzheimer's disease, and central nervous system damage resulting from stroke, ischemia and trauma. Such compositions are useful as anti-inflammatory agents, such as for the treatment of arthritis, with the additional benefit of having significantly less harmful side effects. These compositions are useful in the treatment of allergic rhinitis, respiratory distress syndrome, endotoxin shock syndrome, and atherosclerosis. Such compositions are also useful in the treatment of pain, but not limited to postoperative pain, dental pain, muscular pain, and pain resulting from cancer. Such compositions are useful for the prevention of dementias, such as Alzheimer's disease.

Besides being useful for human treatment, these compositions are also useful for veterinary treatment of companion animals, exotic animals, and farm animals, including mammals, rodents, and the like. More preferred animals include horses, dogs, and cats.

### EXAMPLES

The invention is illustrated to S-[2-[(1-iminoethyl)amino]ethyl]-2-methyl-L-cysteine dihydrochloride, herein identified as "Compound 1 ".

### Example 1

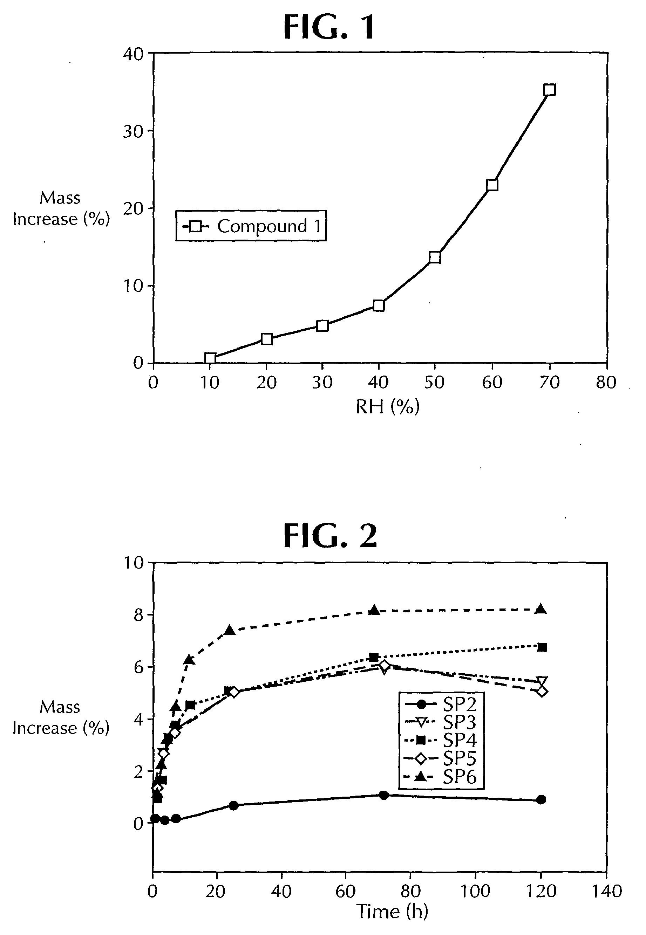

Compound 1 was prepared according to processes described in WO 01/72703. A sample of Compound 1 in substantially dry, amorphous form was subjected to moisture sorption analysis. Dynamic vapor sorption (DVS) was used to determine mass increase (relative to dry mass) was monitored over an adsorption range of 10 - 70% relative humidity (RH) in increments of 10%. As shown by the moisture sorption isotherm represented in Fig. 1, Compound 1 is hygroscopic.

**Table 1. Composition of solid particulate compositions SP1 - SP14**

| **Composition** | **Compound 1 (g)** | **Excipient(s) (g)** |
|---|---|---|
| SP1 | 3 | Microcrystalline cellulose (7) |
| SP2 | 3 | Microcrystalline cellulose (3); |
| | | Hydroxypropyl methylcellose (4) |
| SP3 | 5 | Microcrystalline cellulose (3); |
| | | Hydroxypropyl methylcellose (2) |
| SP4 | 3 | Microcrystalline cellulose (6.95); |
| | | Silicon dioxide (0.05) |
| SP5 | 3 | Hydroxypropyl methylcellose (3) |
| SP6 | 3 | Polyethylene glycol 8000 (5); |
| | | Hydroxypropyl methylcellose (2) |
| SP7 | 3 | Lactose (7) |
| SP8 | 3 | D-Trehalose dihydrate (7) |
| SP9 | 3 | Mannitol (6); |
| | | Hydroxypropyl methylcellose (1) |
| SP10 | 3 | Calcium phosphate tribasic (4) |
| | | Hydroxypropyl methylcellose (3) |
| SP11 | 5 | Calcium phosphate tribasic (3); |
| | | Hydroxypropyl methylcellose (2) |
| SP12 | 3 | Calcium sulfate (4); |
| | | Hydroxypropyl methylcellose (3) |
| SP13 | 3 | Calcium sulfate (4) |
| | | Silicon dioxide (0.05) |
| SP14 | 3 | Corn starch (7) |

### Example 2

Batches of fourteen solid particulate compositions, SP1 - SP14, each having a composition shown in Table 1, were prepared according to the following procedure. Several intermediate solutions were prepared by dissolving Compound 1 in free base form in water at a concentration of 318.4 mg/ml. One or more excipients were added to each of the intermediate solutions to form final solutions. Each final solution was then individually spray dried using a Yamato Pulvis Basic Unit, Model GB-21 (Yamato Scientific America, Inc.) spray dryer under the following conditions: (a) Inlet temperature: 135 °C; (b) Outlet temperature: 75 °C; (c) Atomizing air: 0.75 Kg_{f}/cm²; (d) Drying air: 4 gauge setting; (e) Feed Rate: 5-7 mL/min; (f) Feed total solids concentration: 15% - 21% (wt), to form solid particulate compositions SP1 - SP-14.

### Example 3

Hygroscopicity of solid particulate compositions SP1 - SP14 of Example 2 was assessed according to the following procedure. Samples (40 - 60 mg) of each composition were subjected to controlled environmental conditions of 40% humidity (hygrometer measurement) and ambient temperature (*i.e.* 21 - 23 °C) using a sealed humidity chamber over a saturated potassium carbonate solution (theoretical RH - 44%). At various time points during 120 hours of storage, each sample was weighed and water uptake was determined as the measure of sample mass gain (%). As shown in Figs. 1 and 2, equilibrium water uptake for all compositions tested ranged from 2% - 8%. Compositions SP4, SP7, SP8, SP13 and SP14 formed cake-like, sticky masses within 12 hours of initiation of humidity treatment. Compositions SP2, SP5, SP11, and SP12 remained free-flowing powders throughout the study.

### Example 4

The amount of drug present in each of compositions SP2, SP5, SP6, SP10, SP12 and SP14 of Example 3 was determined using high performance liquid chromatography (HPLC). Data, shown in Table 2, represents an average of 3 to 5 samples; relative standard deviation is also provided for each batch analyzed.

**Table 2. Amount of drug present in SP2, SP5, SP6, SP10, SP12 and SP14**

| **Composition** | **Drug weight (%)** | **Number of Samples** | **Relative Standard Deviation (%)** |
|---|---|---|---|
| SP2 | 32.6 | 5 | 2 |
| SP5 | 39.6 | 5 | 0.5 |
| SP6 | 26.0 | 3 | 3.5 |
| SP10 | 26.2 | 5 | 1.1 |
| SP12 | 26.6 | 5 | 1.0 |
| SP14 | 34.4 | 5 | 4.6 |

These data show that each batch of composition tested exhibited relatively high drug loading and good homogeneity (as shown by low standard deviation).

### Example 5

Three solid particulate compositions, SP15 - SP17, each having a composition shown in Table 2, were prepared according to the following procedure. An intermediate solution was prepared by dissolving Compound 1 (in free base form) in water at a concentration of 318.4 mg/ml. One or more excipients were added to aliquots of the intermediate solution to form several final solutions. Each final solution was then individually spray dried using a Niro Mobile Minor spray dryer under the following conditions: (a) Inlet temperature: 135 °C; (b) Outlet temperature: 75 °C; (c) Atomizing air: 0.75 Kg_{f}/cm²; (d) Drying air: 4 gauge setting; (e) Feed rate: 5 - 7 mL/min; (f) Feed total solids concentration: 15% - 21% (wt) to form solid particulate compositions SP15 - SP17. Three matching placebo compositions, P1 - P3, were prepared according to a substantially similar process.

**Table 3. Composition of solid particulate compositions SP15 - SP17**

| **Composition** | **Compound 1 (g)** | **Excipient(s) (g)** |
|---|---|---|
| SP15 | 30 | Microcrystalline cellulose (40); |
| | | Hydroxypropyl methylcellose (30) |
| SP16 | 35 | Hydroxypropyl methylcellose (35) |
| SP17 | 30 | Hydroxypropyl methylcellose (30); |
| | | Calcium phosphate tribasic (40) |

| **Composition** | **Compound 1 (g)** | **Excipient(s) (g)** |
|---|---|---|
| P1 | 0 | Microcrystalline cellulose (20); |
| | | Hydroxypropyl methylcellose (15) |
| P2 | 0 | Hydroxypropyl methylcellose (360) |
| P3 | 0 | Hydroxypropyl methylcellose (15); |
| | | Calcium phosphate tribasic (20) |

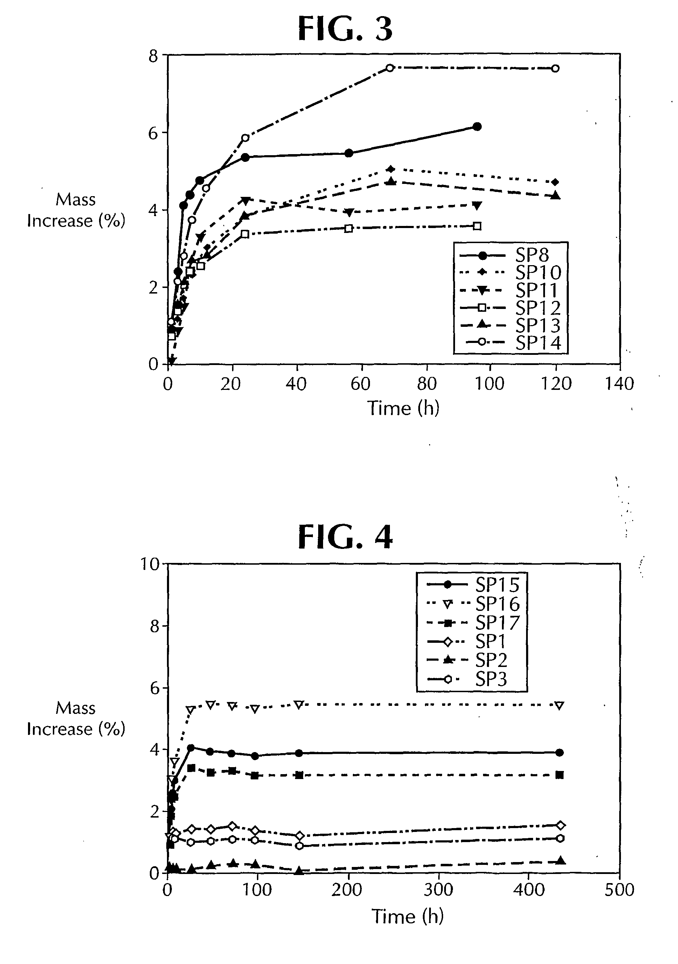

Hygroscopicity profiles for compositions SP15 - SP17 and matching placebo compositions P1 - P3 were determined at 40% RH according to the procedure described in Example 3; data are shown in Fig. 4. While all three placebo compositions exhibited lower equilibrium water uptake than did any of SP15 - SP17, each of SP15 - SP17 exhibited less than 6% water uptake after 400 hours of humidity treatment. These data indicate that the hygroscopicity problem associated with Compound 1 (as seen in Example 1 has been overcome to a surprisingly effective extent.

### Example 6

Five solid particulate compositions, SP18 - SP22, each having a composition shown in Table 4, were prepared according to the following procedure. An intermediate solution was prepared by dissolving Compound 1 (in free base form) in water at a concentration of 318.4 mg/ml. One or more excipients were added to aliquots of the intermediate solution to form several final solutions. Each final solution was then individually spray dried using a Niro Utility Production Minor spray dryer under the following conditions: (a) Batch size: 220 - 420 g; (b) Inlet temperature: 112 - 138 °C; (c) Outlet temperature: 75 °C; (d) Atomizing air: 300 Hz; (f) Feed rate: 100 mL/min; (g) Feed total solids concentration: 30% (wt), to form solid particulate compositions SP18-SP22.

**.Table 4. Composition of solid particulate compositions SP18 - SP23**

| **Composition** | **Compound 1 (g)** | **Excipient(s) (g)** | **Batch Size (g)** |
|---|---|---|---|
| SP18 | 55 | HPMC (55); | 220 |
| | | Microcrystalline cellulose (73); | |
| SP19 | 66 | HPMC (66); | 220 |
| | | Microcrystalline cellulose (44) | |
| SP20 | 66 | HPMC (33); | 220 |
| | | Microcrystalline cellulose (77) | |
| SP21 | 66 | HPMC (17); | 220 |
| | | Microcrystalline cellulose (94) | |
| SP22 | 125 | HPMC (31); | 416 |
| | | Microcrystalline cellulose (177) | |

### Example 7

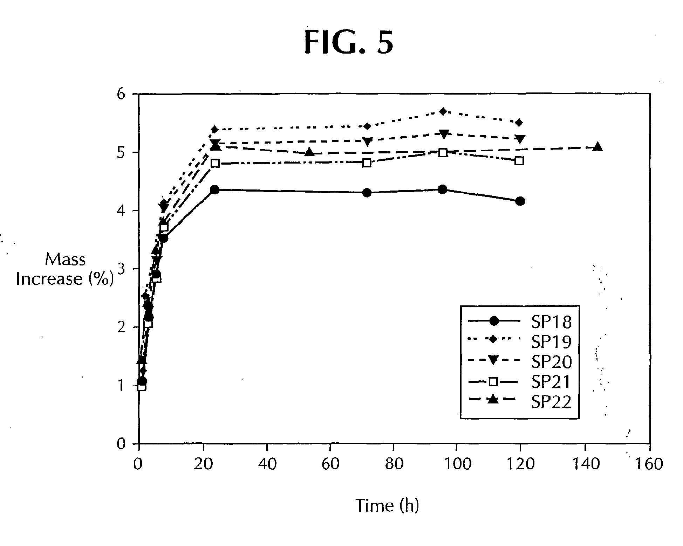

Hygroscopicity profiles for compositions SP18 - SP22 were determined at 40% RH according to the procedure described in Example 3. As shown in Fig. 5, all compositions exhibited less than 6% mass increase throughout humidity treatment. Additionally, compositions SP18 and SP19 remained flowable powders throughout humidity treatment. Compositions SP20 - SP22 exhibited some caking by 24 hours.

### Example 8

Dissolution rate of drug from solid particulate compositions SP15 - SP17 of Example 5 was determined *in vitro.* An amount of composition sufficient to provide a 100 mg dose of drug was individually filled into a size 1 hard gelatin capsule and placed in 900 ml of 0.1 N HCl and was stirred at 50 rpm using a Type II apparatus. Data are shown in Table 5. Overall, all three compositions exhibited dissolution time suitable for use of the compositions in preparing a pharmaceutical dosage form and, if desired, an immediate-release dosage form.

**Table 5. Percent of Compound 1 dissolved from compositions SP15 - SP17 at various times**

| **Time (min.)** | **SP15** | **SP16** | **SP17** |
|---|---|---|---|
| 0 | 0 | 0 | 0 |
| 15 | 60.66 | 91.3 | 45 |
| 30 | 100.6 | 102 | 86.5 |
| 45 | 102.3 | 102 | 102 |
| 60 | 102.3 | 102 | 102 |

## Claims

1. A solid particulate composition comprising a drug selected from S-[2-[(1-iminoethyl)amino]ethyl]-2-methyl-L-cysteine and its dihydrochloride salt; and at least one non-hygroscopic cellulosic polymer, wherein the drug and the at least one non-hygroscopic cellulosic polymer have been first co-dispersed in an aqueous liquid, and subsequently said liquid has been removed, and wherein said composition is non-hygroscopic such that said composition exhibits an increase in mass of not more than 10% when subjected to conditions of 40% relative humidity at a temperature of between 21-23 degrees C for 24 hours.

2. The composition according to claim 1, wherein the at least one non-hygroscopic polymer and the drug are present in a weight ratio of 1:2 to 2:1.

3. The composition according to claim 1 or claim 2, wherein the drug is present in an amount of 10% to 80%, by weight of the composition.

4. The composition according to any preceding claim, wherein the at least one non-hygroscopic polymer is present in a total amount of 10% to 85%, by weight of the composition.

5. The composition according to any preceding claim wherein the at least one non-hygroscopic polymer exhibits a moisture content at 40% relative humidity and 21-23°C of not more than 6%.

6. The composition according to any preceding claim, wherein the at least one non-hygroscopic polymer is selected from the group consisting of hydroxypropylmethylcelluloses, hydroxypropylcelluloses, hydroxyethylcelluloses, methylcelluloses and ethylcelluloses.

7. The composition according to any preceding claim, further comprising a filler.

8. The composition according to Claim 7 wherein the filler is selected from the group consisting of a tribasic calcium phosphate, an anhydrous calcium sulfate, a carboxymethylcellulose calcium, a carboxymethylcellulose sodium, an anhydrous dextrose, a fructose, an anhydrous lactose, an anhydrous magnesium stearate, a magnesium trisilicate, a maltodextrin, a methylcellulose, a microcrystalline cellulose, a powdered cellulose, a pregelatinized starch, a starch, a sterilizable maize starch, a compressible sugar and a confectioner's sugar.

9. The composition according to any preceding claim in the form of a flowable and/or compressible powder.

## Patentansprüche

1. Feste teilchenförmige Zusammensetzung, die einen Arzneistoff, der aus S-[2-[(1-Iminoethyl)amino]ethyl]-2-methyl-L-cystein und dessen Dihydrochloridsalz ausgewählt ist, und mindestens ein nicht-hygroskopisches Cellulosepolymer umfasst, wobei der Arzneistoff und das mindestens eine nicht-hygroskopische Cellulosepolymer zuerst in einer wässrigen Flüssigkeit codispergiert wurden und die Flüssigkeit anschließend entfernt wurde und wobei die Zusammensetzung derart nicht-hygroskopisch ist, dass die Zusammensetzung eine Zunahme der Masse von nicht mehr als 10 % zeigt; wenn sie den Bedingungen einer relativen Luftfeuchtigkeit von 40 % bei einer Temperatur zwischen 21 und 23 °C während 24 h unterzögen wurde.

2. Zusammensetzung nach Anspruch 1, wobei das mindestens eine nicht-hygroskopische Polymer und der Arzneistoff in einem Gewichtsverhältnis von 1:2 bis 2:1 vorhanden sind.

3. Zusammensetzung nach Anspruch 1 oder Anspruch 2, wobei der Arzneistoff in einer Menge von 10 bis 80 Gew.-% der Zusammensetzung vorhanden ist.

4. Zusammensetzung nach einem der vorhergehenden Ansprüche, wobei das mindestens eine nicht-hygroskopische Polymer in einer Gesamtmenge von 10 bis 85 Gew.-% der Zusammensetzung vorhanden ist.

5. Zusammensetzung nach einem der vorhergehenden Ansprüche, wobei das mindestens eine nicht-hygroskopische Polymer einen Feuchtigkeitsgehalt bei einer relativen Luftfeuchtigkeit von 40 % und 21 - 23 °C von nicht mehr als 6 % zeigt.

6. Zusammensetzung nach einem der vorhergehenden Ansprüche, wobei das mindestens eine nicht-hygroskopische Polymer aus der Gruppe von Hydroxypropylmethylcellulosen, Hydroxypropylcellulosen, Hydroxyethylcellulosen, Methylcellulosen und Ethylcellulosen ausgewählt ist.

7. Zusammensetzung nach einem der vorhergehenden Ansprüche, die ferner einen Füllstoff umfasst.

8. Zusammensetzung nach Anspruch 7, wobei der Füllstoff aus der Gruppe von Tricalciumphosphat(V), wasserfreiem Calciumsulfat, Carboxymethylcellulose-calcium, Carboxymethylcellulose-natrium, wasserfreier Dextrose, Fructose, wasserfreier Lactose, wasserfreiem Magnesiumstearat, Magnesiumtrisilicat, Maltodextrin, Methylcellulose, mikrokristalliner Cellulose, pulverförmiger Cellulose, vorgelatinisierter Stärke, Stärke, sterilisierbarer Maisstärke, komprimierbarem Zucker und Staubzucker ausgewählt ist.

9. Zusammensetzung nach einem der vorhergehenden Ansprüche in der Form eines fließfähigen und/oder komprimierbaren Pulvers.

## Revendications

1. Composition en particules solides, comprenant un médicament choisi entre la S-[2-[(1-iminoéthyl)amino]éthyl]-2-méthyl-L-cystéine et son dichlorhydrate; et au moins un polymère cellulosique non hygroscopique, dans laquelle le médicament et ledit au moins un polymère cellulosique non hygroscopique ont été tout d'abord codispersés dans un liquide aqueux, puis ledit liquide a été éliminé, ladite composition étant non hygroscopique de telle sorte que ladite composition présente une augmentation de masse non supérieure à 10 % lorsqu'elle est soumise à des conditions d'humidité relative de 40 % à une température comprise entre 21 et 23°C pendant 24 heures.

2. Composition suivant la revendication 1, dans laquelle ledit au moins un polymère non hygroscopique et le médicament sont présents en un rapport pondéral de 1:2 à 2:1.

3. Composition suivant la revendication 1 ou la revendication 2, dans laquelle le médicament est présent en une quantité de 10 % à 80 % en poids de la composition.

4. Composition suivant l'une quelconque des revendications précédentes, dans laquelle ledit au moins un polymère non hygroscopique est présent en une quantité totale de 10 % à 85 %, en poids de la composition.

5. Composition suivant l'une quelconque des revendications précédentes, dans laquelle ledit au moins un polymère non hygroscopique présente une teneur en humidité, à une humidité relative de 40 %, à une température de 21 à 23°C non supérieure à 6 %.

6. Composition suivant l'une quelconque des revendications précédentes, dans laquelle ledit au moins un polymère non hygroscopique est choisi dans le groupe consistant en des hydroxypropylméthylcelluloses, des hydroxypropylcelluloses, des hydroxyéthylcelluloses, des méthylcelluloses et des éthylcelluloses.

7. Composition suivant l'une quelconque des revendications précédentes, comprenant en outre une charge.

8. Composition suivant la revendication 7, dans laquelle la charge est choisie dans le groupe consistant en phosphate tricalcique, un sulfate de calcium anhydre, une carboxyméthylcellulose calcique, une carboxyméthylcellulose sodique, un dextrose anhydre, un fructose, un lactose anhydre, un stéarate de magnésium anhydre, un trisilicate de magnésium, une maltodextrine, une méthylcellulose, une cellulose microcristalline, une cellulose pulvérisée, un amidon prégélatinisé, un amidon de maïs stérilisable, un sucre compressible et un sucre de confiserie.

9. Composition suivant l'une quelconque des revendications précédentes, sous forme d'une poudre fluide et/ou compressible.
